# EUROPEAN PATENT SPECIFICATION

(11) **EP 0 382 577 B1**
(45) Date of publication and mention of the grant of the patent: **22.05.1996**
(21) Application number: 90301462.9
(22) Date of filing: 12.02.1990
(51) Int. Cl.: C08B 11/20, C08B 15/02, C12P 19/14

(54) **Enzymatic cellulose derivative hydrolysate**
Enzymatisches Hydrolysat von einem Cellulosederivat
Hydrolysat enzymatique d'un dérivé de cellulose

(30) Priority: 10.02.1989 US 309387; 23.06.1989 US 370629
(43) Date of publication of application: 16.08.1990
(73) Proprietor: Alko Group Ltd., FIN-00180 Helsinki (FI)
(72) Inventor: Timonen, Maritta, SF-00101 Helsinki (FI); Turunen,Marja, SF-00101 Helsinki (FI); Vaara,Martti, SF-00101 Helsinki (FI); Vaara, Timo, SF-00101 Helsinki (FI)
(74) Representative: Woods, Geoffrey Corlett

(56) References cited:
- DE-A- 667 864
- FR-A- 1 437 796
- US-A- 4 427 778
- TAPPI (TECHNICAL ASSOCIATION OF THE PULP AND PAPER INDUSTRY), vol. 61, no. 5,1978, pages 101-105, Atlanta, US; H. STEEGE et al.: "Microcrystalline,cellulose powders"

## Description

This invention relates to enzymatic cellulose derivatives hydrolysates and fractions thereof. The invention also provides methods for producing the same, as well as novel uses for the enzymatic hydrolysate and its fractions.

### BACKGROUND OF THE INVENTION

Cellulose derivatives such as carboxymethylcellulose, methylcellulose, methylethylcellulose, hydroxypropylmethylcellulose and hydroxypropylcellulose are non-caloric (non-metabolizable by humans or intestinal flora in human beings), odorless, tasteless water-soluble or water suspendable polymers derived from cellulose. These cellulose derivatives may act as thickeners, binders, stabilizers, suspending agents or flow control agents. They form films resistant to oils, greases and organic solvents. They dissolve rapidly in cold and hot water and are physiologically inert. These functions make them suitable for use in a broad range of applications in food, pharmaceutical, cosmetic, paper and other industries.

For such applications, degradation of cellulose derivatives is normally considered undesirable and to be avoided. Cellulolytic and viscosity reducing enzymes have been deliberately avoided in the past.

Enzymatic hydrolysis of cellulose derivatives have been studied in the past in the context of synergism studies among combinations of enzymes, the possible indexing of substituent distribution patterns, the effect of various substituents on enzymatic hydrolysis and the like. Such studies have been published in the following: Chouchon et al, Biotech. Bioeng., Vol. 26 pp. 988-991 (1984); Henrissat et al, Biotechnology, Vol. 3, pp. 722-726 (1985); Chetkarov et al, Monatshelte Fur Chemie, Vol. 116, pp. 1433-45 (1985); Chetkarov et al, Monatshefte Fur Chemie, Vol. 117, pp. 1021-1026 (1986); Wirick, J. Polym,. Sci., Part A-1, Vol. 6, pp. 1195-1174 (1968); Bhattacharjee, J. Polym. Sci., Part C, Vol. 36, pp. 509-521 (1971). Reduction of chain length determinations have also been studied. Almin et al, Arch. Biochem. Biophys., pp. 124, 129 (1968); Chose, Biotech. Bioeng., Vol. 11, pp.239 (1969).

German patent 667864 describes a process for reducing the viscosity of cellulose ether solutions using enzymes derived from microorganisms.

The present invention deals with a novel enzymatic hydrolysis of cellulose derivatives and products incorporating such hydrolysates. We have also found that some of the properties of these cellulose derivatives can be further improved by enzymatic hydrolysis into low molecular weight polymers or oligomeric mixtures. These enzymatic hydrolysates and fractions (of the total hydrolysate mixture of oligomers into further separated mixtures of oligomers of varying chain length) thereof are more advantageous for several different applications in food, pharmaceutical, paper, cosmetic and textile industries than high molecular weight cellulose derivatives or their unhydrolysed counterparts.

### SUMMARY OF THE INVENTION

The invention discloses a novel soluble enzymatic hydrolysate of different cellulose derivatives as well as its fractions.

The hydrolysate can be made from carboxymethylcellulose. The hydrolysate can be prepared by different modified and unmodified cellulolytic enzymes, the most preferred sources of the enzyme being strains of *Trichoderma reesei* and *Aspergillus.*

The enzymatic hydrolysate and its fractions have several applications in food, paper, pharmaceutical and cosmetic industry, but they are especially useful as calorie saving or low caloric substitutes in a wide range of food stuffs.

In accordance with the invention there is provided a water soluble enzymatic hydrolysate of a cellulose derivative comprising a water-soluble carboxymethylcellulose derivative hydrolysed with a cellulase preparation having endo-1,4-betaglucanase activity to form a mixture of oligomers having an average degree of polymerization in the range of 3 to 300 and a molecular weight of 500 to 100,000 and a viscosity of not more than 20 mPa.s at 25°C at a concentration of 20% by weight, measured with a Haake-Rotovisco viscometer with sensor systems NV. The enzyme preparation is selected from the group of cellulases, modified cellulases and mixtures thereof.

The enzyme preparation is a cellulase or modified cellulase (i.e. modified to remove or prevent the formation of saccharide producing enzymes in the cellulase preparation in the first instance, e.g., by genetic alteration of the microorganism from which the cellulase preparation is prepared) preferably produced from microorganisms selected from the group of *Trichoderma, Aspergillus* and *Penicillium.* Most preferably the cellulase preparation is derived from *Trichoderma reesei* from which at least one of beta-glucosidase and cellobiohydrolase activities have been removed. The enzyme preparation comprises endo- 1,4-betaglucanase.

The invention also provides a method for producing a water-soluble mixture of oligomers from cellulose derivatives comprising the steps of: selecting a water-soluble carboxymethylcellulose derivative; selecting a cellulolytic material including a cellulase having endo-1,4-betaglucanase activity which hydrolyses the selected cellulose derivative into a mixture of oligomers having an average degree of polymerization in the range of 3 to 300 and a molecular weight in the range of 500 to 100,000 and a viscosity of not more than 20 mPa.s at 25°C at a concentration of 20% by weight, measured with a Haake-Rotovisco viscometer with sensor systems NV; and reacting the selected enzyme with the selected cellulose derivative for a time and at a temperature sufficient to produce the mixture of oligomers.

The step of selecting the cellulase typically comprises selecting a microorganism which produces the cellulase and preparing the cellulase from a culture of the microorganism. The cellulase produced by the microorganism may be purified to remove enzymes which will react with the cellulose derivative to produce saccharides. The microorganism is preferably selected from the group of Trichoderma, Aspergillus and Penicillium.

In order to prevent hydrolysis of the cellulose derivative into saccharides the selected microorganism may alternatively be treated to alter the genes of the microorganism such that production of saccharide generating enzymes by the genes is disenabled.

The invention further contemplates removing all or a portion, typically up to 50% by weight of a selected fat contained in a foodstuff and substituting a mixture of oligomers produced according to the invention for the removed fat; and/or removing up to about 40% of a selected carbohydrate contained in a foodstuff and substituting a mixture of oligomers produced according to the invention for the removed carbohydrate.

The invention also contemplates separating a mixture of oligomers produced according to the invention into fractions of oligomers of different average molecular weight, removing up to 50% by weight of a selected fat contained in a foodstuff, and substituting one or more of the fractions for the removed fat; and/or, separating the mixture of oligomers produced according to the invention into fractions of oligomers different average molecular weight, removing up to 40% by weight of a selected carbohydrate contained in a foodstuff and substituting one or more of the fractions for the removed carbohydrate.

### BRIEF DESCRIPTION OF THE DRAWINGS

FIG. 1 shows molecular weight distribution patterns of a carboxymethylcellulose and its hydrolysate as described in Example 2 (i) herein;
FIG. 2 shows molecular weight distribution patterns of a carboxymethylcellulose and its hydrolysate as described in Example 3 herein;
FIG. 3 shows molecular weight distribution patterns of selected fractions of the carboxymethylcellulose hydrolysate as described in Example 3 herein.

### DETAILED DESCRIPTION OF THE INVENTION

This invention relates to a water-soluble hydrolysate of a cellulose derivative and its fractions. The hydrolysates are characterized by having an average degree of polymerization (DP) in the range of 3-300 and a molecular weight in the range of 500 - 100,000, and a viscosity of not more than 20 mPa.s at 25°C at a concentration of 20 % by weight, measured in a Haake Rotoviscometer with sensor systems NV.

### Enzyme Preparation

Enzymes used in this invention are various food-grade cellulase preparations. They can be produced from a multitude of different microorganisms such as strains of Trichoderma, Aspergillus, Penicillium, etc. The selected microorganism strain is grown by conventional means in a medium containing food grade materials such that the cellulases are produced, the microorganism is separated from the medium, the medium is collected and typically concentrated and dried. These enzymes can be used as such or in mixtures and they can be modified in many different ways known to the man skilled in the art. The most preferred enzyme preparation is produced from Trichoderma reesei, from which preparations the beta-glucosidase and/or the cellobiohydrolase activities are removed chromatographically or genetically. Beta-glucosidase and/or cellobiohydrolase activities are removed from the selected cellulase preparation so as to prevent the degradation of the cellulose derivative into cellobiose and/or glucose. Genetic alteration of the appropriate enzyme producing microorganism may be effected with radiation or mutagenic chemical agents (or by gene inactivation by recombinant DNA methods) so as to disenable production of beta-glucosidase and cellobiohydrolase by the microorganism. Cellulase preparations suitable for use herein are, e.g., the commercially available cellulase preparations designated as the Econase CE series as produced by Alko Ltd., Helsinki Finland.

### Starting Materials

According to the invention the cellulose derivative hydrolysate and its fractions can be produced by enzymatic hydrolysis of a water-soluble carboxymethyl cellulose derivative.

### General preparation of the hydrolysate

Cellulose derivative hydrolysates are prepared from water-soluble cellulose derivatives as defined above by an enzymatic hydrolysis utilizing a cellulase preparation having endo- 1, 4-beta-glucanase as the sole active hydrolytic agent such that only insignificant amount of saccharides (e.g., glucose and cellobiose) which are absorbed in human intestine (e.g., glucose) or hydrolyzed by the intestinal bacterial flora (e.g., cellobiose)), are produced. On the other hand the average degree of polymerization (DP) of the oligomers formed by such a hydrolysis is lower than 300, and thus the viscosity of solutions of the hydrolysate is reduced significantly compared to the viscosity of solutions of the unhydrolysed cellulose derivatives. The specific conditions suitable for and the specific time sufficient to secure the desired hydrolysis may be readily determined for each selected cellulose derivative and each selected enzyme preparation.

### Use of the cellulose derivative hydrolysates

The cellulose derivatives used as starting materials in the present invention are as such non-caloric. Because the hydrolysis according to the present invention does not produce significant amounts of metabolizable sugars, the hydrolysates according to this invention with their improved properties, are especially useful as low-caloric substitutes in food stuffs.

The cellulose derivative hydrolysates and fractions thereof produced according to this invention can be used for example as new low-caloric fat sparing agents or bulking agents. These hydrolysates can be used to replace fat in varying food stuffs, like baked goods, butter icing and custard. Fat can be replaced by these hydrolysates up to a level of at least 50%, but a level of 40% is most preferred. The amount which can be replaced depends on the application. The texture of the food stuff and the eating quality of the new product should be improved or remain unchanged.

The cellulose derivative hydrolysates and fractions thereof produced according to this invention can be used also as new low-caloric bulking agents. These hydrolysates can be used to replace carbohydrates such as sugar in different kinds of baked products or in other food stuffs. The amount of carbohydrate replaced with these hydrolysates depends on the application and average chain length of the hydrolysate oligomers. By conventional means hydrolysate mixture may be further separated into fractions of oligomers of differing average chain lengths. The viscosity of the various fractions will vary with the degree of average chain length of the oligomers contained within in a fraction. Depending on the particular food stuff application, the invention contemplates selecting a fraction from a hydrolysate mixture having a viscosity (average chain length) which is most appropriate for the particular food stuff application. The selection of a particular average chain length fraction and the amount of such a fraction to be used in any given food stuff application may vary according to the amount of fat or carbohydrate to be replaced, it being recognized that the higher the absolute amount of substitution agent desired to be used in a particular foodstuff, the lower the viscosity (average molecular weight) of the fraction of mixture of oligomers should be used.

The invention is described in greater detail in the following examples.

### Example 1 -- Cellulase Preparation

The beta-glucosidase activity was removed by ion exchange chromatography from the cellulase preparation, Econase CE, Alko Ltd., Helsinki, Finland which was produced from a strain of Trichoderma reesei. The cellulase preparation (column A, Table 1) was passed through a cation exchange column (S-Sepharose FF, Pharmacia, LKB Biotechnology AB, Uppsala, Sweden) and equilibrated with 50 mM sodium acetate pH 3.8 equilibrium buffer. The unbound protein (including oligomer producing endoglucanases) was washed out with the equilibration buffer (column B, Table 1). Beta-glucosidase activity remained bound to the column and could be separately eluted with 1M NaCl.

**TABLE 1**

| Enzyme | Relative Enzyme Activity (%) | |
|---|---|---|
| | A | B |
| | before ion exchange procedure | after ion exchange procedure |
| Beta-glucosidase | 100 | 1 |
| endo-1, 4, -beta-glucanase | 100 | 70 |

Endo- 1, 4- beta-glucanase and beta-glucosidase activities were measured as described by Bailey & Nevalainen (1981): Enzyme Microb. Technol. 3: 153-157. The relative enzyme activities reported in Table 1 of the Econase preparations before and after passage through an ion exchange column demonstrate the results of a typical means according to the invention of preparing an essentially beta-glucosidase free preparation for use in producing the oligomeric hydrolysates contemplated by the invention.

Although Table 1 reports relative enzyme activities, the absolute amount of enzyme used in any particular example is hereafter reported in terms of the amount of enzyme activity of the enzyme employed according to the universal activity unit of nano-katal (nkat) which stands for that amount of enzyme which produces one nanomole of reaction product in one second. (In the context of this application a hydrolysate reaction product such as an oligomer or glucose which is capable of reducing an agent such as dinitrosalicylic acid which is reduced by the hydrolysate reaction product and subsequently measured.) The method of Bailey et. al., Enzyme Microb. Technol., Vol. 9, pp. 153-157 describes how such measurements of enzyme activity can be made using glucose as a standard.

### EXAMPLE 2

### Carboxymethylcellulose hydrolysate

### (i) Hydrolysis with Trichoderma reesei derived enzyme preparation

20 g of carboxymethylcellulose (CMC 7MFD-type, a cellulose gum, also designated by the tradename Blanose and available from Hercules Chemical Company, 92507, Rueil-Malmaison Ceder, France; 7MFD designating a medium viscosity, food grade sodium carboxymethylcellulose having 7 out of 10 glucose units substituted with carboxymethyl) was mixed in 320 1 of water and the pH of the solution was adjusted to 5.5 with 15% phosphoric acid and the temperature was raised to 40°C. 0.27 l of the enzyme preparation having an endo-1, 4 beta-glucanase activity of 1,780,000 nkat from which the beta-glucosidase activity was removed chromatographically (as described in Example 1) was added to the CMC solution. After one hour another 20 kg of CMC was added to the solution. After hydrolysis of 23 hours the enzyme was inactivated by heating (90°C, 15 min). Finally, the hydrolysis solution was concentrated by conventional evaporating and spray-drying.

The product contained less than 2% by weight of glucose and cellobiose. When the same hydrolysis was carried out with the original cellulase enzyme preparation of *Trichoderma reesei*-fungus, the amount of produced glucose and cellobiose was above 5% by weight.

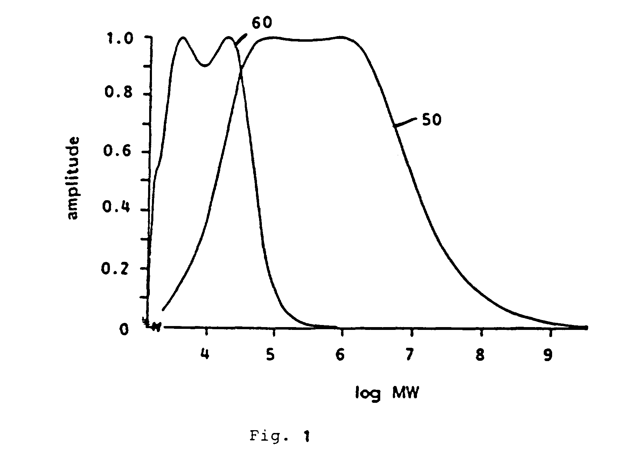

The molecular weight distribution patterns of carboxymethylcellulose, curve 50, and its hydrolysate, curve 60, are shown in Figure 1.

The molecular weight distribution pattern was determined by HPLC using a gel filtration column (TSK gel G2500PW, Toyo Soda Manufacturing Co., Ltd., Japan) with a refractive index detector (HP 1037 A) and Pharmacosmos Dextran Standards (Pharmacosmos, DK-4130, Viby Sj., Denmark). The eluent was 0.5 M sodium chloride.

### (ii) Hydrolysis with Aspergillus and Penicillium derived enzyme preparations

The enzyme preparations selected were commercially available Cellulase Application 3 (Amano Pharmaceutical Co., Ltd., Nagoya, Japan) produced using *Aspergillus* strain and Cellulase CP (Sturge Enzymes, North Yorkshire, England) produced using a *Penicillium* strain. Carboxymethylcellulose hydrolysates were prepared as described in Example 2(i), except that 30g of CMC-7MFD was used in 1 1 of water, and the amounts of enzymes added were 0.028 g of Cellulase AP 3 (having an endo-1, 4 beta-glucanase activity of 1350 nkat) and 0.048 g of Cellulase CP (having an endo-1, 4 beta-glucanase activity of 1350 nkat). The viscosities and molecular weight distributions of the hydrolysates produced by either cellulase were similar (FIG. 1) to the hydrolysate produced with enzymes derived from Trichoderma reesei.

The viscosities of the various cellulose derivatives and their hydrolysates as described and prepared in Example 2 were measured using a Haake-Rotovisco viscometer with sensor systems NV (Karlsruhe, Federal Republic of Germany) (Table 2). The viscosities were measured in water solutions at 25°C. Table 2 set forth the concentrations (by weight) of a variety of solutions all having the same viscosity.

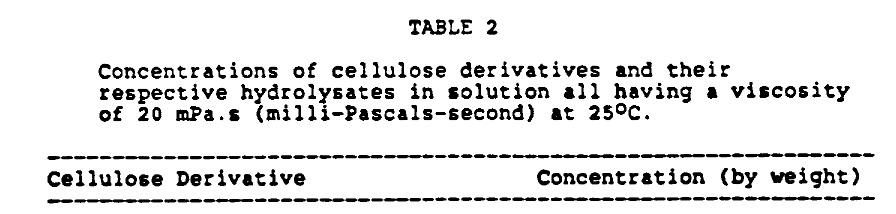

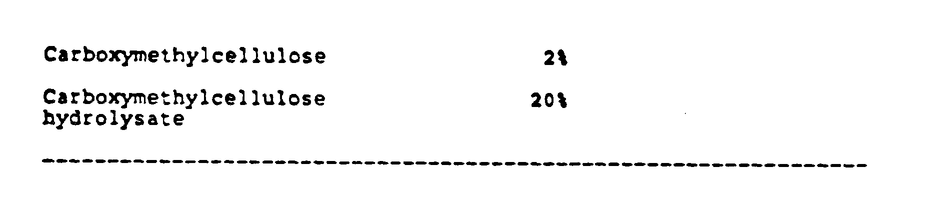

As the data in Table 2 indicate, the hydrolysate of a cellulose derivative has a substantially lower viscosity than an equal amount by weight in aqueous solution of the cellulose derivative itself and can be incorporated into a foodstuff in substantially higher quantity as a fat or sugar substitute than the cellulose derivative itself without compromising the texture, volume, density or the like of the foodstuff.

### Example 3 -- The fractionation of carboxymethylcellulose hydrolysate

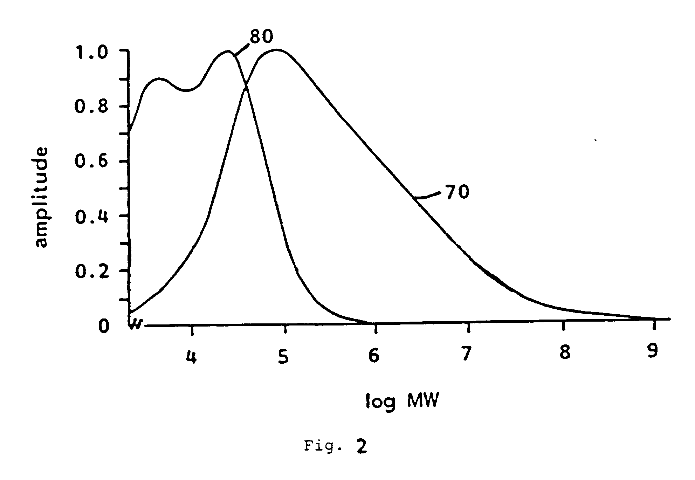

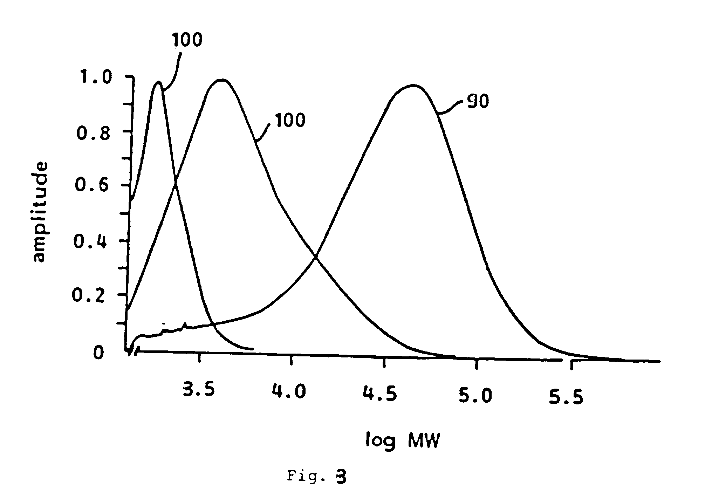

The carboxymethylcellulose hydrolysate was prepared as described in Example 2, except that the raw material was CMC 7LFD designating a low viscosity, food grade cellulose gum having 7 out of 10 glucose units substituted with carboxymethyl, (designated under the tradename Blanose and available from Hercules Chemical Co., France) 1.6 kg CMC was used in 8 l of water and that the amount of enzyme added was 13.2 ml having an endo- 1, 4 beta-glucanase activity of 87,000 nkat. 5 ml of the hydrolysate (0.5 g of dry matter) was further fractionated into three fractions by gel permeation chromatography (Pharmacia K 26/100 -column, Sephacryl S-200 -gel, Pharmacia LKB Biotechnology AB, S-75182 Uppsala, Sweden). The eluent was distilled water, the flow rate was 14 ml/hour, and the fractionation process was carried out for 45 hours and fractions collected at intervals of 0.5 hours and pooled into three fractions (18 hours - 26 hours, curve 90. 26 hours - 32 hours, curve 100, and 32 hours - 38 hours, curve 110, FIG. 3, respectively). The molecular weight distributions of carboxymethylcellulose, curve 70, carboxymethylcellulose hydrolysate, curve 80, and the three further fractions, curves 90, 100, 110, FIGS. 2, 3 were determined by HPLC as described in Example 2.

### Example 4 -- The evaluation of the fat sparing agent according to the invention in Madeira cake

Standard Madeira cakes were prepared by mixing 200 g of high ratio cake flour, 250 g of sugar-caster, 130 g of high ratio shortening 16 g of skimmed milk powder, 3 g of salt, 12 g of baking powder, 180 g of water and 176 g of egg (defrosted). 180 g of the mix was scaled into greased tins and baked at 170°C 30 min. Madeira cakes, in which 40% of the fat was substituted (i.e., 130 g of shortening was reduced by 40% to 78 g) with 1) carboxymethylcellulose hydrolysate (fat sparing agent according to the invention), 2) carboxymethylcellulose or 3) potato maltodextrin (a conventional fat sparing agent) were compared to the standard Madiera cakes and to each other.

According to a trained panel the results were as described in Table 3.

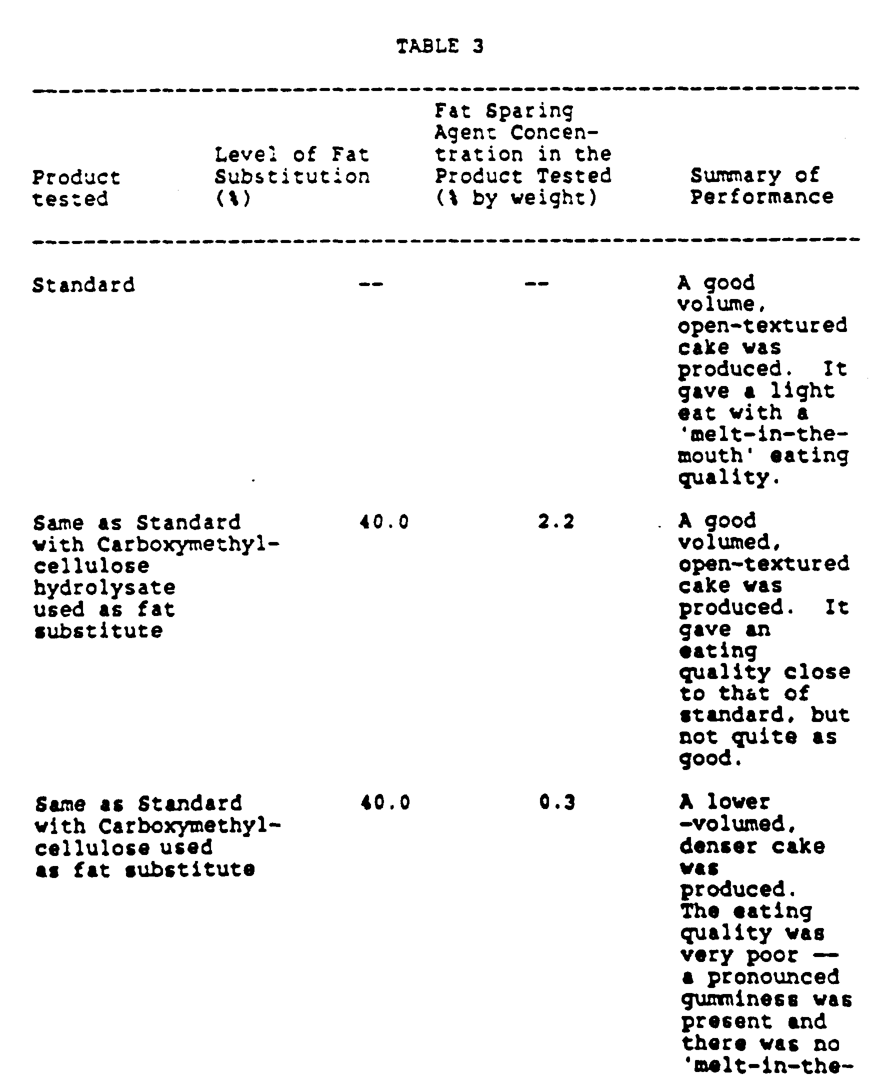

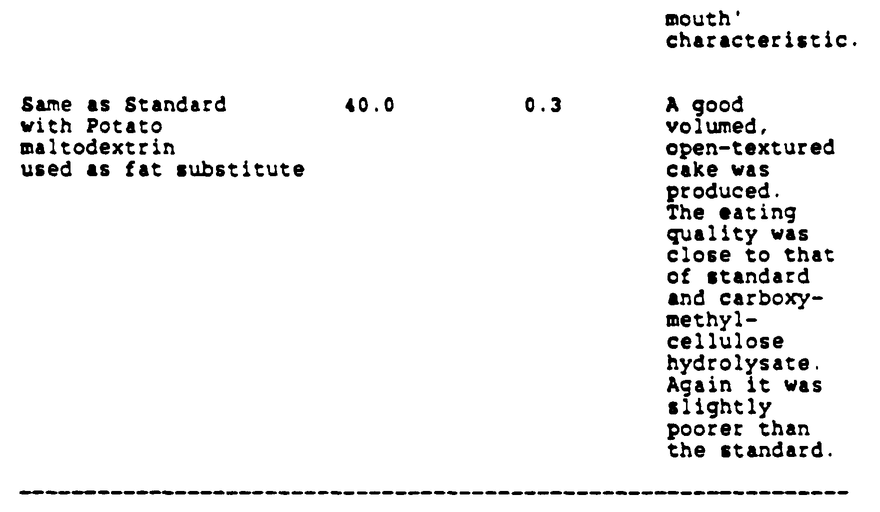

### Example 5 -- Evaluation of the fat sparing agent according to the invention in butter icing

A standard butter icing was prepared by mixing 179 g of butter (unsalted), 225 g of icing sugar and 96 g of water. The butter icings, in which 30% of fat was substituted (i.e., 179 g of butter was reduced by 30% to 125.3 g) with 1) carboxymethylcellulose hydrolysate (fat sparing agent according to this invention), 2) carboxymethylcellulose or 3) potato maltodextrin and all were compared to the standard butter icing and to each other.

According to a trained panel the results were as described in Table 4.

### Example 6 -- Evaluation of the bulking agent according to the invention in marzipan

A standard marzipan product was prepared by mixing 21.8 g of icing sugar, 21.8 g of caster sugar, 43.7 g of ground almonds, 0.8 g of vanilla flavoring, 10.9 g of eggs (lightly beaten) and 1 g of lemon juice for every 100 g of product. The mixture was formed into a ball, kneaded lightly, rolled out and cut out. Marzipan, in which 10% of sugar was substituted with carboxymethylcellulose hydrolysate (bulking agent according to the invention), had good almond flavor and same color as the standard marzipan product. It was also easy to roll and cut out. Higher percentage sugar replacement may be required to reduce calories significantly. Appropriate lower viscosity DP-fractions of carboxymethylcellulose hydrolysate may be employed in greater than 10% by weight amounts, preferably up to as much as about 40% while still maintaining normal texture.

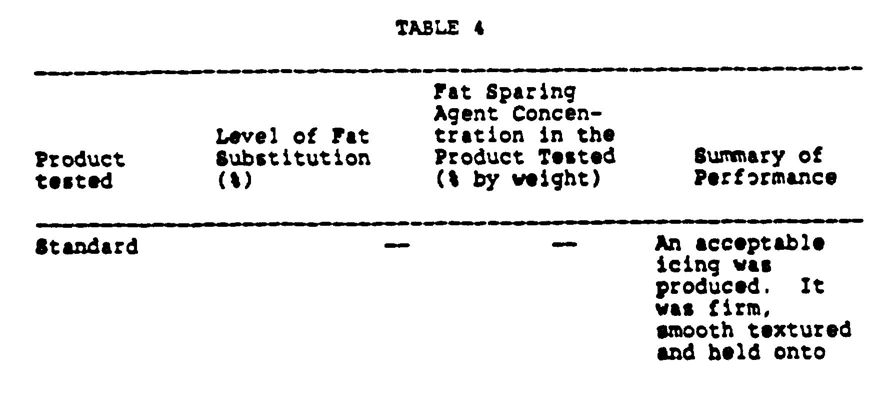

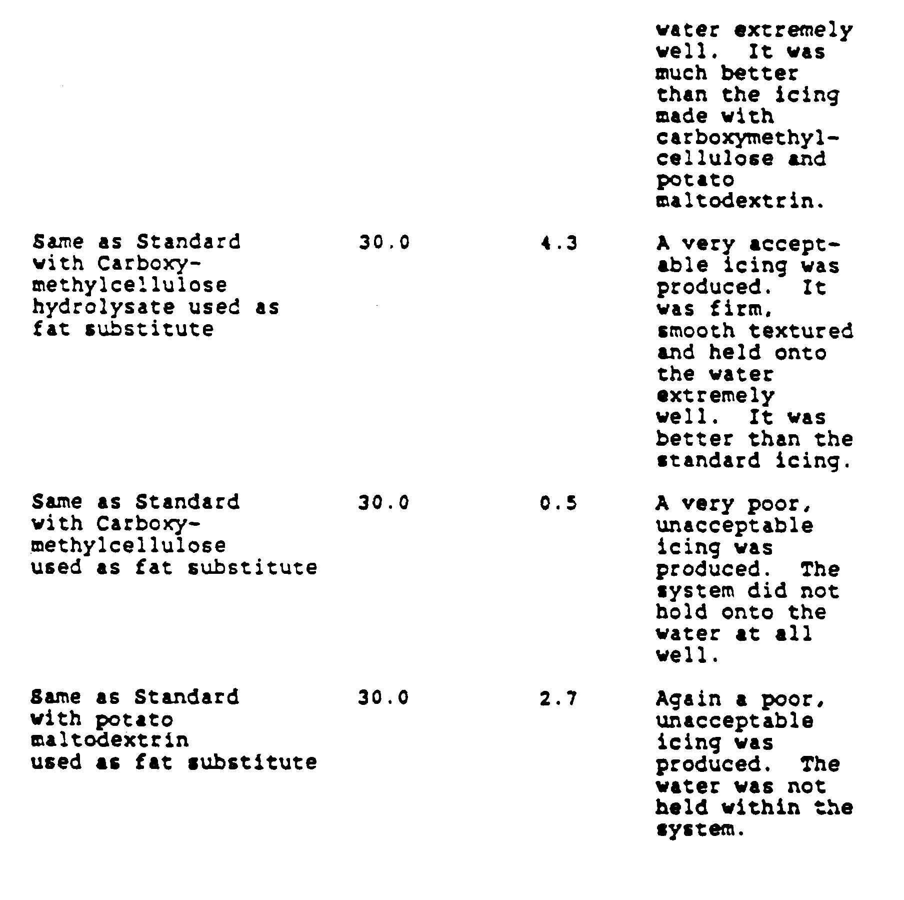

## Claims

1. A water-soluble enzymatic hydrolysate of a cellulose derivative comprising a water-soluble carboxymethylcellulose hydrolysed with a cellulase preparation having endo-1,4-betaglucanase activity to form a mixture of oligomers having an average degree of polymerization in the range of 3 to 300, a molecular weight of 500 to 100,000, and a viscosity of not more than 20 mPa.s at 25°C at a concentration of 20% by weight, measured with a Haake-Rotovisco viscometer with sensor systems NV.

2. An enzymatic hydrolysate according to claim 1 wherein the enzyme preparation is a cellulase or modified cellulase produced from Trichoderma, Aspergillus or Penicillium microorganisms.

3. An enzymatic hydrolysate according to claim 1, wherein the enzyme preparation comprises a cellulase preparation derived from Trichoderma reesei from which at least one of beta-glucosidase and cellobiohydrolase activities have been removed.

4. A method for producing a water-soluble mixture of oligomers from cellulose derivatives which comprises hydrolysing a water-soluble carboxymethyl cellulose derivative with a cellulolytic material including a cellulase having endo-1,4-betaglucanase activity which hydrolyses the selected cellulose derivative into a mixture of oligomers having an average degree of polymerization in the range of 3 to 300, a molecular weight in the range of 500 to 100,000, and a viscosity of not more than 20 mPa.s at 25°C at a concentration of 20% by weight, measured with a Haake-Rotovisco viscometer with sensor systems NV, for a time and at a temperature sufficient to produce the mixture of oligomers.

5. A method according to claim 4 wherein the cellulolytic material used is prepared from a culture of a microorganism.

6. A method according to claim 5 wherein the microorganism is Trichoderma, Aspergillus or Penicillium.

7. A method according to claim 5 or 6 wherein the cellulolytic material produced by the microorganism is purified to remove enzymes which react with the cellulose derivative to produce saccharides.

8. A method according to claim 5 or 6 wherein the selected microorganism is treated to alter the genes of the microorganism such that production of saccharide generating enzymes by the genes is disenabled.

## Patentansprüche

1. Wasserlösliches enzymatisches Hydrolysat eines Cellulosederivats, das eine wasserlösliche Carboxymethylcellulose enthält, die mit einer Cellulasepräparation mit endo-1,4-beta-Glucanase-Aktivität unter Bildung einer Mischung aus Oligomeren mit einem mittleren Polymerisationsgrad im Bereich von 3 bis 300, einem Molekulargewicht von 500 bis 100 000 und einer Viskosität von nicht mehr als 20 mPa·s bei 25°C und einer Konzentration von 20 Gew.-%, gemessen mit einem Haake-Rotovisco-Viskosimeter mit NV-Sensorsystemen, hydrolysiert ist.

2. Enzymatisches Hydrolysat nach Anspruch 1, worin die Enzympräparation eine Cellulase oder eine modifizierte Cellulase ist, die aus Trichoderma-, Aspergillus- oder Penicillium-Mikroorganismen hergestellt ist.

3. Enzymatisches Hydrolysat nach Anspruch 1, worin die Enzympräparation eine Cellulasepräparation umfaßt, die aus Trichoderma reesei stammt, aus der mindestens eine beta-Glucosidase- und Cellobiohydrolase-Aktivität entfernt worden ist.

4. Verfahren zur Herstellung einer wasserlöslichen Oligomermischung aus Cellulosederivaten, bei dem während eines Zeitraums und bei einer Temperatur, die zur Herstellung der Oligomermischung ausreichend sind, ein wasserlösliches Carboxymethylcellulose-Derivat mit einem cellulolytischen Material hydrolysiert wird, das eine Cellulase mit endo-1,4-beta-Glucanase-Aktivität umfaßt, welche das betreffende Cellulosederivat zu einer Oligomermischung mit einem mittleren Polymerisationsgrad im Bereich von 3 bis 300, einem Molekulargewicht im Bereich von 500 bis 100 000 und einer Viskosität von nicht mehr als 20 mPa·s bei 25°C und einer Konzentration von 20 Gew.-%, gemessen mit einem Haake-Rotovisco-Viskosimeter mit NV-Sensorsystemen, hydrolysiert.

5. Verfahren nach Anspruch 4, worin das verwendete cellulolytische Material aus einer Kultur eines Mikroorganismus hergestellt wird.

6. Verfahren nach Anspruch 5, worin als Mikroorganismus Trichoderma, Aspergillus oder Penicillium verwendet wird.

7. Verfahren nach Anspruch 5 oder 6, worin das durch den Mikroorganismus hergestellte cellulolytische Material zur Entfernung von Enzymen, die mit dem Cellulosederivat unter Bildung von Sacchariden reagieren, gereinigt wird.

8. Verfahren nach Anspruch 5 oder 6, worin der betreffende Mikroorganismus zur Veränderung der Gene des Mikroorganismus in der Weise behandelt wird, daß die Produktion von Saccharid erzeugenden Enzymen durch die Gene ausgeschaltet wird.

## Revendications

1. Hydrolysat enzymatique soluble dans l'eau d'un dérivé de cellulose comprenant une carboxyméthylcellulose soluble dans l'eau hydrolysée avec une préparation de cellulase ayant une activité d'endo-1,4-β-glucanase pour former un mélange d'oligomères ayant un degré moyen de polymérisation situé dans la gamme de 3 à 300, et un poids moléculaire de 500 à 100 000, et une viscosité qui n'est pas supérieure à 20 mPa.s à 25°C à une concentration de 20 % en poids, mesurée à l'aide d'un viscosimètre Haake-Rotovisco avec des systèmes de détection NV.

2. Hydrolysat enzymatique selon la revendication 1 dans lequel la préparation enzymatique est une cellulase ou une cellulase modifiée produite par des microorganismes Trichoderma, Aspergillus ou Penicillium.

3. Hydrolysat enzymatique selon la revendication 1, dans lequel la préparation enzymatique comprend une préparation de cellulase dérivée de Trichoderma reesei de laquelle on a supprimé au moins une des activités de β-glucosidase et de cellobiohydrolase.

4. Méthode de production d'un mélange soluble dans l'eau d'oligomères à partir de dérivés de cellulose qui comprend l'étape consistant à hydrolyser un dérivé de carboxyméthylcellulose soluble dans l'eau avec une substance cellulolytique incluant une cellulase ayant une activité d'endo-1,4-β-glucanase qui hydrolyse le dérivé de cellulose choisi en un mélange d'oligomères ayant un degré moyen de polymérisation situé dans la gamme de 3 à 300, un poids moléculaire dans la gamme de 500 à 100 000, et une viscosité qui n'est pas supérieure à 20 mPa.s à 25°C à une concentration de 20 % en poids, mesurée à l'aide d'un viscosimètre Haake-Rotovisco avec des systèmes de détection NV, pendant un temps et à une température suffisants pour produire le mélange d'oligomères.

5. Méthode selon la revendication 4 dans laquelle la substance cellulolytique utilisée est préparée à partir d'une culture d'un microorganisme.

6. Méthode selon la revendication 5 dans laquelle le microorganisme est Trichoderma, Aspergillus ou Penicillium.

7. Méthode selon la revendication 5 ou 6 dans laquelle la substance cellulolytique produite par le microorganisme est purifiée afin d'éliminer les enzymes qui réagissent avec le dérivé de cellulose pour produire des saccharides.

8. Méthode selon la revendication 5 ou 6 dans laquelle le microorganisme choisi est traité pour modifier les gènes du microorganisme de façon à inactiver les gènes des enzymes générant des saccharides.
